**Europäisches Patentamt**

**European Patent Office**

**Office européen des brevets**

(19)

(11) Veröffentlichungsnummer: **0 338 243 B1**

(12) # EUROPÄISCHE PATENTSCHRIFT

(45) Veröffentlichungstag der Patentschrift :
22.05.91 Patentblatt 91/21·

(51) Int. Cl.$^5$ : **B26B 13/20**

(21) Anmeldenummer : **89104371.3**

(22) Anmeldetag : **11.03.89**

(54) **Schere, insbesondere Friseurschere.**

(30) Priorität : **20.04.88 DE 3813273**

(43) Veröffentlichungstag der Anmeldung :
**25.10.89 Patentblatt 89/43**

(45) Bekanntmachung des Hinweises auf die
Patenterteilung :
**22.05.91 Patentblatt 91/21**

(84) Benannte Vertragsstaaten :
**DE ES FR GB IT**

(56) Entgegenhaltungen :
**DE-A- 2 811 398
DE-U- 8 801 248
US-A- 3 906 630
US-A- 4 742 617**

(73) Patentinhaber : **" JAGUAR"
STAHLWARENFABRIK GMBH & CO. KG
22, Ketzberger Strasse
W-5650 Solingen 16 (DE)**

(72) Erfinder : **Pracht, Günther
17, Nettelbeckstrasse
W-5650 Solingen (DE)**

(74) Vertreter : **Lippert, Hans-Joachim, Dipl.-Ing. et
al
Patentanwälte Dipl.-Ing. W. Dahlke, Dipl.-Ing.
H.-J. Lippert Birkenweiher 15
W-5650 Solingen (DE)**

EP 0 338 243 B1

**Beschreibung**

Die Erfindung betrifft eine Schere, insbesondere eine Friseurschere, bei der vornehmlich das für sich hergestellte, an der beweglichen Scherenhälfte befindliche, zur Aufnahme des Daumens des die Schere Handhabenden dienende Griffauge begrenzt schwenkbar ist, gemäß Oberbegriff der Ansprüche 1 bzw. 3.

Derartige Scheren sind bekannt. Sie haben gegenüber Scheren, bei denen das Griffauge der beweglichen Scherenhälfte fest angeordnet ist, den Vorteil, daß sich beim Hindurchstecken des Daumens das Griffauge mit seiner Innenfläche satt an den Daumen anlegt und während des Betätigens der Schere in dieser Lage verbleibt. Dies hat zur Folge, daß selbst bei längerem, anhaltendem Gebrauch der Schere keine Druckstellen mehr an dem Daumen entstehen und Verspannungen im Handgelenk vermieden werden.

Bei einer gattungsgemäßen bekannten Schere (DE-C 28 11 398) ist das schwenkbare Griffauge in der Nut eines aus synthetischem Werkstoff bestehenden Halmauges so gelagert, daß es sich beim Hindurchstecken des Daumens satt an diesem anlegt. Das Halmauge weist dabei einen hohlzylindrischen Ansatz auf, mit dem es auf dem Halm der beweglichen Scherenhälfte fest aufgesetzt ist.

Derartige Scheren haben einen wesentlichen Nachteil, der in folgendem zu erblicken ist. Es hat sich in der Praxis ergeben, daß sich das Halmauge während der Benutzung aufweitet, so daß eine einwandfreie Lagerung des Griffauges nicht mehr gewährleistet ist. Es hat sich ferner ergeben, daß der Bereich, in dem das Griffauge verschwenkbar ist, häufig zu klein ist. Dies hat zur Folge, daß sich der Daumen beim Verschwenken des Griffauges an dem inneren Rand des Halmauges reibt, was bei längerem, anhaltendem Gebrauch einer Friseurschere als unangenehm empfunden wird. Die Führung des verschwenkbaren Griffauges in der Nut des Halmauges ist aber eine zwingende Notwendigkeit, da sich anderenfalls das Griffauge unbeabsichtigt um 360° verschwenken könnte. Dies würde sich aber bei einer Friseurschere in folgendem Falle nachteilig auswirken. Der Friseur hält bei bestimmten Arbeiten, bei denen er in kurzen Zeitabständen abwechselnd Schere und Kamm benötigt, die Schere nur mit dem Ringfinger, um mit Daumen, Zeige- und Mittelfinger den Kamm aufzunehmen und handhaben zu können. Nach Ablegen des Kammes würde aber das Hindurchstecken des Daumens durch das verschwenkbare Griffauge insofern erschwert, als das Griffauge erst auf den Daumen eingestellt werden müßte. Hieran ändert auch nichts der Umstand, daß das schwenkbare Griffauge am äußeren Umfang einen Kugelkopf aufweist, mit dem es in einer Pfanne des Halmes der beweglichen Scherenhälfte gelagert ist.

Bei einer anderen bekannten Friseurschere (DE-PS 29 24 830) ist der an dem beweglichen Griffauge befindliche Kugelkopf mit einem aus flexiblem Werkstoff bestehenden Ring in der Pfanne des Kugelkopfes gesichert, wobei die Pfanne auf einem fest auf dem Halm aufgesetzten zylindrischen Ansatz angeformt ist. Das schwenkbare Griffauge ist unter Fortfall des bisherigen Halmauges unbegrenzt verschwenkbar, wobei durch den Ring freies Schwenken des Griffauges verhütet ist, weil auf das Griffauge zum Verschwenken ein leichter Fingerdruck ausgeübt werden muß.

Den beiden bekannten Scheren ist jedoch ein Nachteil gemeinsam, der darin zu erblicken ist, daß die Schere durch das Halmauge bzw. durch die Pfanne für den Kugelkopf auffallend von der herkömmlichen Form einer Friseurschere abweicht, insbesondere klobig wirkt, wodurch die bekannten Scheren trotz ihrer Vorzüge gegenüber den herkömmlichen Scheren von einem beachtlichen Teil der Friseure nicht benutzt werden. Die klobige Gestaltung löst des weiteren bei den Friseuren die Vermutung aus, daß die Scheren relativ schwer im Gewicht sind und ihre Handhabung beeinträchtigt ist. Der Friseur bevorzugt daher die herkömmlichen Scheren, die in ihrer Gesamtheit aus Metall oder aber einem Werkstoff bestehen, der die Beibehaltung der herkömmlichen Gestalt der Scheren ermöglicht.

Es ist Aufgabe der Erfindung, die Lagerung des zur Aufnahme des Daumens bestimmten beweglichen Griffauges der beweglichen Scherenhälfte einer Schere der eingangs genannten Gattung zu vereinfachen und zu verbessern, und zwar unter weitgehender Beibehaltung der herkömmlichen Form der Friseurscheren.

Die Lösung der Aufgabe besteht nach einer Ausführung der Erfindung in den Merkmalen gemäß Anspruch 1.

Das schwenkbare Griffauge gemäß der Erfindung ist in einem Halmauge auf zwei Zapfen gelagert, deren Achse schräg zur Scherenmittellinie angeordnet ist.

Die erfindungsgemäße Anbringung des beweglichen Griffauges ermöglicht es, die Scherenhälften für sich einstückig herzustellen, wobei in vorteilhafter Weise die Form der herkömmlichen Friseurscheren beibehalten wird. Die Begrenzung des Winkelbereiches, in dem das bewegliche Griffauge verschwenkbar ist, kann dabei auf einfache Weise dadurch erreicht werden, daß die Wandung des beweglichen Griffauges breiter ausgeführt ist als die des Halmauges, wodurch das bewegliche Griffauge am Ende der Verschwenkung an die Innenwandung des Halmauges anschlägt.

Ausgehend von einer Schere der eingangs angegebenen Gattung, insbesondere einer Friseurschere, mit einem längeren Halm, einem Fingergriffauge und einer an diesem angebrachten Fingerstütze sowie mit einem kürzeren Halm und einem schwenkbaren Griffauge ist nach der weiteren Erfindung das Griffauge am kürzeren Halm lösbar angeordnet und in einer zur Scherenmittellinie schräggestellten Achse schwenkbar gelagert. Hierdurch ist es in vorteilhafter Weise möglich, zur Anpassung an verschiedene Daumengrößen jeweils das passende Griffauge am beweglichen, kürzeren Halm zu befestigen und ggf. wieder zu lösen, wobei die Schwenkbarkeit beibehalten ist.

Bei einer veränderten Ausführung der Erfindung ist am kürzeren Halm ein Halmauge mit zwei innen angebrachten Zapfen befestigt, die in einer Achse schräg zur Scherenmittellinie liegen und in Bohrungen des schwenkbaren Griffauges eingreifen. Für diese Ausführung gelten die gleichen bereits vorstehend angegebenen Vorteile. Dabei kann die Wandung des schwenkbaren Griffauges vorzugsweise breiter sein als die Wandung des Halmauges, wodurch das Schwenken des Griffauges auf den Bereich beschränkt wird, der für eine ergonomisch optimale Handhabung der Schere erforderlich ist.

Eine andere Ausführung der Erfindung besteht in den Merkmalen gemäß Anspruch 3.

Hierbei wird ein Halmauge an der beweglichen Scherenhälfte nicht benötigt, da vorteilhaft der Zapfen des schwenkbaren Griffauges in einer Bohrung des Halmes lösbar gelagert ist. Auch das Auswechseln des schwenkbaren Griffauges zur Anpassung an einen größeren oder kleineren Daumen ist nun durch das Lösen bzw. Befestigen von nur einer Federscheibe wesentlich erleichtert.

Ausgehend von der Scherenmittellinie, die eine objektiv geeignete Bezugslinie ist, beträgt der Winkel zur schräggestellten Achse des schwenkbaren Griffauges je nach Ausführung der Schere 30-60° und wird bei einer bevorzugten Ausführungsform 53° betragen. Der Winkel zwischen der Mittellinie des kürzeren Halmes bzw. der Längsachse des Halmauges und der schrägen Achse des schwenkbaren Griffauges ist in Abhängigkeit von der Spreizung der Halme wesentlich kleiner und beträgt 5-30°. Dieser Winkel nimmt zu, wenn die Spreizung der Halme abnimmt.

Das für den Daumen bestimmte schwenkbare Griffauge darf sich nicht ganz frei um die Schwenkachse herum bewegen, damit die Schere durch den Handhabenden, insbesondere einen Friseur, auch dann leicht mit einer Hand gefaßt werden kann, wenn mit der anderen Hand beispielsweise ein Kamm gehalten wird. Die Begrenzung des Schwenkwinkels mit einfachen Maßnahmen ist daher ein weiteres Ziel der Erfindung.

Bei einer gemäß der Erfindung ausgebildeten Schere, insbesondere einer Friseurschere, bei der in einer Abbiegung etwa am Ende des kürzeren Halmes eine Bohrung unter einem Winkel von 30 - 60° schräg zur Scherenmittellinie angeordnet ist, in die ein am schwenkbaren Griffauge angebrachter Zapfen eingesetzt und gegen unbeabsichtigtes Lösen, zum Beispiel mit einem Federring gesichert ist, sind vorteilhaft am Griffauge ein Vorsprung mit einer schrägen Kante und am Ende der Abbiegung des Halmes eine etwa gerade Kante derart angebracht, daß nach dem Schwenken des Griffauges um etwa 50° die Kante des Vorsprunges an der anderen Kante des Halmes zur Anlage kommt. Bei einer anderen vorteilhaften Ausführung zur Begrenzung des Schwenkwinkels des beweglichen Griffauges auf einen ergonomisch günstigen Schwenkbereich von 0 bis etwa 50° besteht darin, daß das Griffauge neben dem Zapfen und dem Ende der Abbiegung gegenüberliegend mit einem Ansatz sowie andererseits der Halm an der dem Griffauge zugekehrten Seite der Abbiegung mit einem Ansatz versehen ist, wobei diese Ansätze an der einander bzw. dem Zapfen zugekehrten Seite mit etwa geraden Kanten derart versehen sind, daß sie einen Schwenkwinkel von ca. 50° zulassen. Diese Ausbildung der Ansätze kann in einfacher Weise zum Beispiel durch Fräsen der einander gegenüberliegenden Flächen am Griffauge und am Ende des kürzeren Halmes hergestellt werden.

Die vorstehend geschilderten und weitere Einzelheiten der Erfindung sind nachstehend anhand der Zeichnungen näher erläutert. Es zeigen :

Fig. 1 eine Friseurschere geschlossen in Vorderansicht,

Fig. 2 in Seitenansicht,

Fig. 3 das Halmauge mit dem beweglichen Griffauge in der Lage, als wäre der Daumen hindurchgesteckt, bei geöffneter Friseurschere,

Fig. 4 einen Schnitt in vergrößertem Maßstab nach der Linie IV-IV der Fig. 1,

Fig. 5 eine vereinfachte Ausführung einer Friseurschere, ähnlich Fig. 1, jedoch ohne Halmauge,

Fig. 6 eine Einzelheit bei "B" der Fig. 5 vergrößert,

Fig. 7 die Einzelheit "B" in Richtung des Pfeiles "C" der Fig. 6 betrachtet,

Fig. 8 eine Einzelheit bei "D" der Fig. 5 vergrößert in gegenüber Fig. 6 veränderter Ausführung und

Fig. 9 einen Schnitt entlang der Schnittlinie IX-IX der Fig. 8.

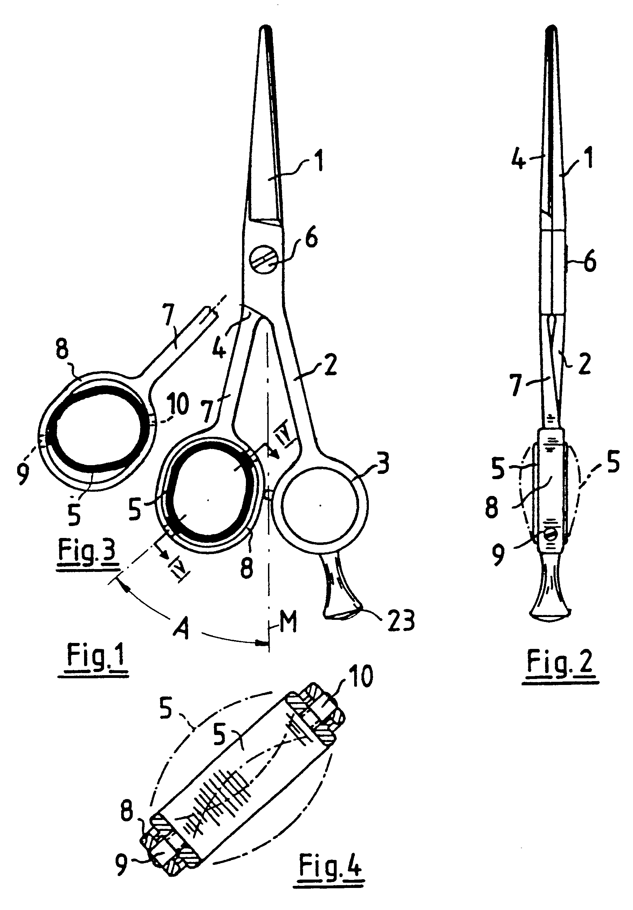

Die in den Fig. 1-4 dargestellte Friseurschere besteht aus der bei der Handhabung feststehenden Scherenhälfte 1 mit dem am Halm 2 befindlichen Griffauge 3 mit Fingerstütze 23 und der beweglichen Scherenhälfte 4 mit dem schwenkbaren Griffauge 5. Die Scherenhälften 1, 4 sind durch eine Gelenkschraube 6 miteinander verbunden. Am Halm 7 der beweglichen Scherenhälfte 4 ist ein mit dem Halm 7 einstückiges Halmauge 8 angeordnet, in dem das schwenkbare Griffauge 5 gelagert ist. Das Griffauge 5 ist auf zwei an der Innenwandung des Halmauges 8 beiderseits der Längsachse des letzteren fest angeordneten Zapfen 9, 10 gelagert, die so schräg zueinander angeordnet sind, daß sich das Griffauge 5 mit seiner Innenfläche beim Hindurchstecken des Daumens nach Öffnen der Friseurschere satt an den Daumen anlegt. Die Wandung des Griffauges 5 ist zur Begrenzung der Verschwenkbarkeit um ein geringes Maß breiter als die Wandung des Halmauges 8 (Fig. 2 und 4).

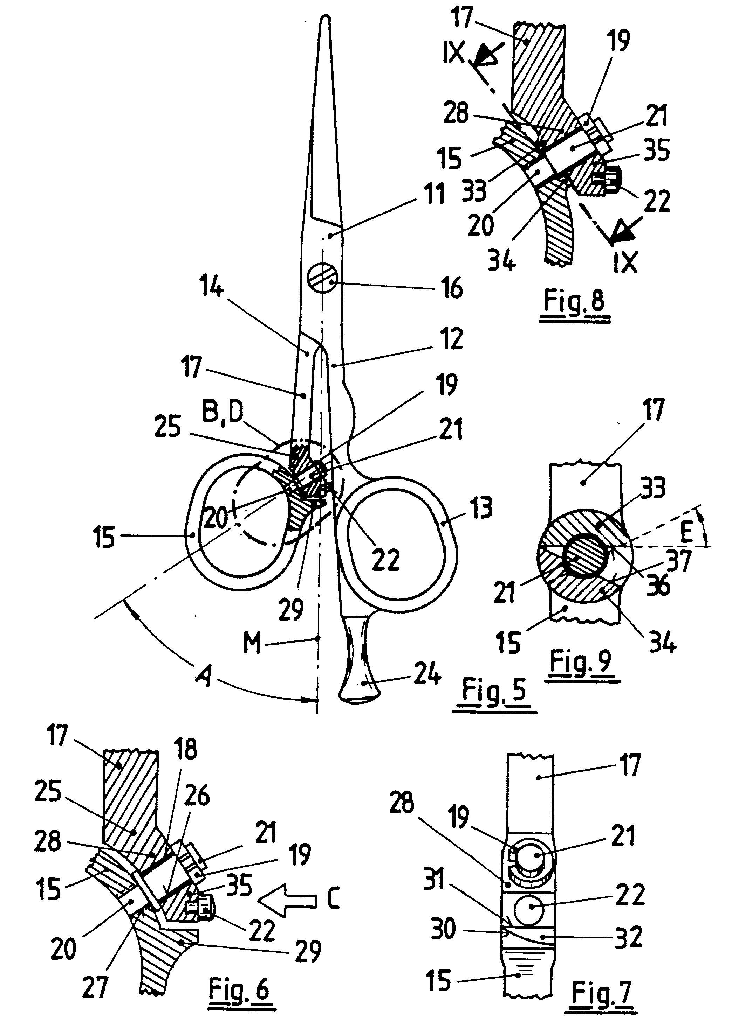

Die in den Fig. 5-7 gezeigte Friseurschere besteht aus der bei der Handhabung festliegenden Scherenhälfte 11, mit am Halm 12 befestigtem Griffauge 13 mit Fingerstütze 24 und der durch den Daumen bewegbaren Scherenhälfte 14 mit dem schwenkbaren Griffauge 15. Die Scherenhälften sind durch eine Gelenkschraube 16 miteinander verbunden. Am Ende 25 weist der Halm 17 der beweglichen Scherenhälfte 14 zum Halm 12 der anderen Scherenhälfte 11 hin eine Abbiegung 28 auf, die mit einer Bohrung 26 für einen Zapfen 21 (Fig. 6) versehen ist. Das Zapfenende 20 ist in einer Gewindebohrung 27 des Griffauges 15 festgeschraubt. Der Zapfen 21 steht zur Scherenmittellinie M unter einem Winkel A, der zwischen 30-60° liegen kann und bevorzugt 53° beträgt. Nach Einschrauben des Zapfens 21 in die Gewindebohrung 27 des Griffauges 15 wird eine Kunststoffbuchse 18 auf den Mantel des Zapfens 21 geschoben und nach dem Einsetzen in die Bohrung 26 wird der Zapfen 21 mit einem Federring 19 gegen unbeabsichtigtes Lösen am Ende 25 des Halmes 17 gesichert. An der Abbiegung 28 ist zum Halm 12 hin ein elastischer Puffer 22 angebracht, der das metallische Zusammenschlagen der Halme 12 und 17 verhindert. Zwischen der Kante 30 eines am Griffauge 15 angeordneten Vorsprunges 29 und der Kante 31 der Abbiegung 28 ist ein winkelförmiger Zwischenraum 32 vorgesehen. Beim Verschwenken aus der in Fig. 7 dargestellten Nullage während der Handhabung der Schere kann das Griffauge 15 auf dem Zapfen 21 bis zum Anschlag der Kanten 30 und 31 in einem Schwenkbereich von 5-50° gedreht werden. Dies ist der ergonomisch günstigste Bereich. Die Begrenzung des Schwenkwinkels erleichtert das Erfassen der Schere, insbesondere des Griffauges 15 mit dem Daumen des Handhabenden, da ein Schrägstellen des Griffauges 15 in einem Drehbereich, der nicht vom Daumen erfaßt werden kann, vermieden wird.

Eine andere vorteilhafte Ausführung zur Begrenzung des Schwenkwinkels des beweglichen Griffauges 15 im Schwenkbereich von 0 bis eta 50° besteht nach den Fig. 8 und 9 darin, daß das Griffauge 15 neben dem Zapfen 21 und dem Ende 35 der Abbiegung 28 gegenüberliegend mit einem Ansatz 34 sowie der Halm 17 an dem Griffauge 15 zugekehrten Seite der Abbiegung 28 mit einem Ansatz 33 versehen ist. Diese Ansätze 33 und 34 sind an der dem Zapfen 21 zugewandten Seite mit etwa geraden Kanten 36 und 37 derart versehen, daß sie einen Schwenkwinkel E von ca. 50° zwischen dem Griffauge 15 und dem Halm 17 zulassen. Diese geraden Kanten 36,37 lassen sich in einfacher Weise, zum Beispiel durch Fräsen, herstellen.

Bezugszeichenliste

1 Scherenhälfte, feststehende
2 Halm (von 1)
3 Griffauge (von 1)
4 Scherenhälfte, bewegliche
5 Griffauge (von 4, für Daumen)
6 Gelenkschraube
7 Halm (von 4)
8 Halmauge (von 4)
9 Zapfen (in 8)
10 Zapfen (in 8)
11 Scherenhälfte, festehende, Fig. 5-7
12 Halm (von 11)
13 (Finger-)Griffauge (an 12)
14 Scherenhälfte, bewegliche
15 Griffauge (von 14, für Daumen)
16 Gelenkschraube
17 Halm von (14)
18 Kunststoffbuchse (für 20)
19 Federring (für 20)
20 Zapfenende (in 15)
21 Zapfen (in 17)
22 Puffer (an 17, gegen 12)
23 Fingerstütze (an 3)
24 Fingerstütze (an 13)
25 Ende (von 17)
26 Bohrung (in 25)
27 Gewindebohrung (in 15)
28 Abbiegung (an 17)
29 Vorsprung (an 15)
30 Kante (an 29)
31 Kante (an 35)
32 Zwischenraum (zwischen 30 und 31)
33 Ansatz (an 28)
34 Ansatz (an 15)
35 Ende (von 28)
36 Kante (an 33)
37 Kante (an 34)
A Winkel (zwischen Scherenmittellinie und Achse des Zapfens 21)
B Einzelheit (in Fig. 5, vergrößert dargestellt in Fig. 6)
C Pfeil (in Fig. 6)
M Scherenmittellinie

**Ansprüche**

1. Schere, insbesondere Friseurschere, mit einem längeren Halm, einem Fingergriffauge sowie mit einem kürzeren Halm und einem schwenkbaren Griffauge, wobei das schwenkbare Griffauge in einer zur Scherenmittellinie schräg gestellten Achse schwenkbar gelagert ist, dadurch gekennzeichnet, daß am kürzeren Halm (7) ein Harmauge (8) mit zwei innen angebrachten Zapfen (9, 10) befestigt ist, die in einer Achse schräg zur Scherenmittellinie (M) liegen und in Bohrungen des schwenkbaren Griffauges (5) eingreifen.

2. Schere nach Anspruch 1, dadurch gekennzeichnet, daß die Wandung des schwenkbaren Griffauges (5) breiter ist als die Wandung des Halmauges (8).

3. Schere, insbesondere Friseurschere, mit einem längeren Halm, einem Fingergriffauge sowie mit einem kürzeren Halm und einem schwenkbaren Griffauge, wobei das schwenkbare Griffauge in einer zur Scherenmittellinie schräg gestellten Achse schwenkbar gelagert ist, dadurch gekennzeichnet, daß etwa am Ende (25) des kürzeren Halmes (17) in einer zum längeren Harm (12) hin weisenden Abbiegung (28) eine Bohrung (26) unter einem Winker von 30 bis 60° schräg zur Scherenmittellinie (M) angeordnet ist, in die ein am schwenkbaren Griffauge (15) angebrachter Zapfen (21) eingesetzt und gegen unbeabsichtigtes Lösen mit einem Federring (19) gesichert ist.

4. Schere, nach einem der Ansprüche 1-3, dadurch gekennzeichnet, daß das schwenkbare Griffauge (5, 75) am kürzeren Halm (7, 17) in einer um 30 bis 60°, insbesondere um 53°, zur Scherenmittellinie (M) schräggestellten Achse schwenkbar gelagert ist.

5. Schere, nach einem der Ansprüche 3 oder 4, dadurch gekennzeichnet, daß am Griffauge (15) ein Vorsprung (29) mit einer schrägen Kante (30) und am Ende (35) der Abbiegung (28) des Halmes (17) eine gerade Kante (31) derart angebracht sind, daß nach dem Schwenken des Griffauges (15) um etwa 50° die Kante (30) das Vorsprunges (29) an der anderen Kante (31) des Halmes (17) zur Anlage kommt.

6. Schere, nach einem der Ansprüche 3 oder 4, dadurch gekennzeichnet, daß am Griffauge (15) neben dem Zapfen (21) ein Ansatz (34) mit einer schrägen Kante (37) und an der Abbiegung (28) des Halmes (17) ein Ansatz (33) mit einer Kante (36) derart angebracht sind, daß nach dem Schwenken des Griffauges (15) um etwa 50° die Kante (37) des Ansatzes (34) an der anderen Kante (36) des Ansatzes (33) zur Anlage kommt.

## Claims

1. Scissors, particularly for use by barbers, with a longer handle, a finger loop and with a shorter handle and a pivotable finger loop, whereby the pivotable finger loop is pivotably arranged in an oblique axis relative to the centre line of the scissors, characterized in that a handle ring (8) ist mounted at the shorter handle (7), the handle ring (8) having two pins (9, 10) mounted inside, which are arranged in an axis oblique relative to the centre line (M) of the scissors and which engage in bores at the pivotable finger loop (5).

2. Scissors as claimed in claim 1, characterized in that the wall of the pivotable finger loop (5) is broader than the wall of the handle ring (8).

3. Scissors, particularly for use by barbers, with a longer handle, a finger loop and with a shorter handle and a pivotable finger loop, whereby the pivotable finger loop is pivotably arranged in an oblique axis relative to the centre line of the scissors, characterized in that approximately at the end (25) of the shorter handle (17) in a bent portion (28), which is directed towards the longer handle (12), there is arranged a bore (26), extending obliquely at an angle of 30° to 60° relative to the centre line (M) of the scissors, a pin (21) fastened to the pivotable finger loop (15) and being secured against unintentional loosening by means of a spring washer (19) being inserted into that bore.

4. Scissors as claimed in anyone of claims 1 to 3, characterized in that the pivotable finger loop (5, 15) at the shorter handle (7, 17) is pivotably mounted in an axis extending obliquely at an angle of 30° to 60°, preferably 53°, relative to the centre line (M) of the scissors.

5. Scissors as claimed in claim 3 or 4, characterized in that a projection (29) having an inclined edge (30) is arranged at the finger loop (15) and that at the end (35) of the bent portion (28) of the handle (17) there is arranged a straight edge (31) in such a manner that after pivoting the finger loop (15) by about 50° the edge (30) of the projection (29) engages the other edge (31) of the handle (17).

6. Scissors as claimed in claim 3 or 4, characterized in that at the finger loop (15) adjacent to the pin (21) there is arranged a projection (34) having an inclined edge (37) and at the bent portion (28) of the handle (17) there is arranged a projection (33) having an edge (36) in such a manner that, after pivoting the finger loop (15) by about 50°, the edge (37) of the projection (34) abuts the other edge (36) of the projection (33).

## Revendications

1. Ciseaux, particulièrement pour coiffeurs, avec une branche plus longue, un anneau à queue ainsi qu'avec une branche plus courte et un anneau intérieur basculant, l'anneau intérieur basculant étant logé de façon à pouvoir basculer dans un axe oblique par rapport à l'axe de ciseaux, ciseaux caractérisés par ce qu'à la branche plus courte (7) est fixé un anneau de branche (8) ayant deux tenons (9, 10) disposés à l'intérieur qui se trouvent sur un axe oblique par rapport à l'axe de ciseaux (M) et qui prennent dans des trous de l'anneau intérieur basculant (5).

2. Ciseaux suivant revendication 1, caractérisés par ce que la paroi de l'anneau intérieur basculant (5) est plus large que la paroi de l'anneau de branche (8).

3. Ciseaux, particulièrement pour coiffeurs, avec une branche plus longue, un anneau à queue ainsi qu'avec une branche plus courte et un anneau intérieur basculant, l'anneau intérieur basculant étant logé de façon à pouvoir basculer dans un axe oblique par rapport à l'axe de ciseaux, ciseaux caractérisés par ce qu'approximativement à l'extrémité (25) de la branche plus courte (17), dans une partie pliée (28) orientée vers la branche plus longue (12), est disposé un trou (26) sous un angle de 30 à 60° obliquement par rapport à l'axe de ciseaux (M), dans lequel trou est insérée une cheville (21) fixée à l'anneau intérieur basculant (15) et assurée par un circlip contre le desserrage inopiné.

4. Ciseaux suivant une des revendications 1 à 3, caractérisés par ce que l'anneau intérieur basculant (5, 15) à la branche plus courte (7, 17) est logé de façon à pouvoir basculer dans un axe oblique de 30 à 60°, particulièrement de 53°, par rapport à l'axe de ciseaux (M).

5. Ciseaux suivant une des revendications 3 ou 4, caractérisés par ce qu'à l'anneau (15) sont disposés un ergot (29) pourvu d'un chant oblique (30) et, à l'extrémité (35) de la partie pliée (28) de la branche (17), un chant droit (31) et cela de telle façon qu'après le basculement de l'anneau (15) d'environ 50°, le chant (30) de l'ergot (29) vient s'appuyer contre l'autre chant (31) de la branche (17).

6. Ciseaux suivant une des revendications 3 ou 4, caractérisés par ce qu'à l'anneau (15) sont disposés, à côté de la cheville (21), un épaulement (34) pourvu d'un chant oblique (37) et, à la partie pliée (28) de la branche (17), un épaulement (33) pourvu d'un chant (36) et cela de telle façon qu'après le basculement de l'anneau (15) d'environ 50°, le chant (37) de l'épaulement (34) vient s'appuyer contre l'autre chant (36) de l'épaulement (33).

1

6

7

8

10

9

5

5

4

7

2

3

23

M

A

IV

IV

Fig.3

Fig.1

4   1

6

7   2

5   5

8

9

Fig.2

5

5

10

8

9

Fig.4

Fig. 8

Fig. 5

Fig. 9

Fig. 6

Fig. 7